(19) Europäisches Patentamt
European Patent Office
Office européen des brevets

(11) **EP 3 395 232 A1**

(12) **EUROPÄISCHE PATENTANMELDUNG**

(43) Veröffentlichungstag:
**31.10.2018   Patentblatt 2018/44**

(51) Int Cl.:
*A61B 3/00* (2006.01)

(21) Anmeldenummer: **17168314.7**

(22) Anmeldetag: **26.04.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **PHARMPUR GmbH**
**86343 Königsbrunn (DE)**

(72) Erfinder:
• LINGENFELDER, Christian
  89081 Ulm (DE)
• HESSLING, Martin
  89075 Ulm (DE)
• KÖLBL, Philipp
  89077 Ulm (DE)

(74) Vertreter: **Leißler-Gerstl, Gabriele**
**Hoefer & Partner**
**Patentanwälte mbB**
**Pilgersheimer Strasse 20**
**81543 München (DE)**

(54)   **VORRICHTUNG ZUR BELEUCHTUNG DES INTRAOKULAREN RAUMES**

(57)   Es wird eine Vorrichtung zur gezielten Beleuchtung eines intraokularen Raumes eines menschlichen oder tierischen Auges beschrieben, die a) einen Sockel (2) mit einem proximalen Ende und einem distalen Ende, b) einen Schaft (3), mit einem proximalen Ende und einem distalen Ende, c) eine Sonde (4), mit einem proximalen Ende und einem distalen Ende, d) eine Lichtquelle (5) mit mindestens einer LED, und e) eine mit der Lichtquelle (5) verbundene Energiequelle (6) umfasst, wobei der Schaft an seinem distalen Ende mit dem proximalen Ende des Sockels verbindbar ist, die Sonde am proximalen Ende mit dem distalen Ende des Sockels verbindbar ist und wobei die Lichtquelle (5) am distalen Ende der Sonde (4) positioniert ist.

Figur 1

EP 3 395 232 A1

**Beschreibung**

**Gebiet der Erfindung**

Die Erfindung betrifft eine Vorrichtung zur Illumination des Auges gemäß dem Oberbegriff des Anspruchs 1.

**Hintergrund der Erfindung**

Endoilluminatoren zur Ausleuchtung des Auges sind bekannt, weisen jedoch Nachteile auf. So ist z.B. aus US 2004/0004846 A1 eine Vorrichtung bekannt, die in einem Gehäuse eine LED als Lichtquelle aufweist, wobei diese Lichtquelle Licht in eine Lichtfaser einspeist, die dann das Auge ausleuchtet. Nachteil einer derartigen Vorrichtung ist es, dass in solchen Vorrichtungen das Licht nur sehr ineffizient von der Lichtquelle zum zu beleuchtenden Ort gebracht wird, sodass am vorgesehenen Ort nur etwa 1% der Lichtleistung ankommt.

Obwohl eine gute Ausleuchtung des Auges bzw. des Augenhintergrundes notwendig ist, muss auch darauf geachtet werden, dass keine phototoxischen Schäden hervorgerufen werden. Helles und energiereiches Licht kann Schäden an der Netzhaut, den Photorezeptoren und dem Sehnerv hervorrufen. So ist zwar bläuliches Licht für Operationen bevorzugt, da die Umgebung im Auge rötlich ist und blaues Licht für bessere Wahrnehmung sorgt, bläuliches Licht ist jedoch energiereicheres Licht und kann daher eher zu Schäden führen. Mit anderen Worten ist daher eine sehr gute Ausleuchtung eines Operationsbereiches erwünscht, zugleich soll jedoch die Ausleuchtung möglichst schonend für das Auge im Hinblick auf die Strahlenbelastung, aber auch im Hinblick auf das Ausmaß der Intervention am Auge erfolgen. Bekannte Vorrichtungen, die mit Lichtleitern arbeiten, vermeiden zwar in der Regel eine hohe Strahlenbelastung des Auges, führen aber zu einer geringeren Ausleuchtung. Eine hohe Strahlenbelastung entsteht insbesondere, wenn die Lichtquelle zu nahe an die Netzhaut kommt und/oder wenn die Beleuchtung zu lange dauert. Die DIN-Norm ISO/DIS15004-2 zeigt die Anforderungen für Behandlungen am und im Auge. Wünschenswert ist daher auch eine Lichtquelle, die ausreichend den gewünschten Bereich ausleuchtet auch wenn sie in einem Abstand dazu gehalten wird.

Endoilluminatoren werden generell in der Ophthalmochirurgie und insbesondere in der Pars-plana-Vitrektomie eingesetzt, um den intraokularen Raum auszuleuchten. Die Nachteile der Endoilluminatoren aus dem Stand der Technik sind insbesondere in der entweder zu geringen Ausleuchtung oder in der hohen Strahlenbelastung des Auges zu sehen.

Es war daher eine Aufgabe der Erfindung, eine Vorrichtung zur Illumination für die Ophthalmochirurgie, z.B. Pars-plana-Vitrektomie bereitzustellen, die das Operationsgebiet gezielt und optimal ausleuchten kann und die empfindlichen Bereiche des Auges, insbesondere die Netzhaut bestmöglich schützt. Weiterhin war es eine Aufgabe der Erfindung, eine Vorrichtung bereitzustellen, die flexibel einstellbar ist, bei der z.B. Farbe, Intensität, Richtung und Abstrahlwinkel des Lichts angepasst werden können und die ein Ausleuchten mit unterschiedlichen Wellenlängen bzw. aus einer Kombination von Wellenlängen ermöglicht. Eine weitere Aufgabe der Erfindung war es, eine Vorrichtung bereitzustellen, die kompakt ist und auch unter schwierigen äußeren Umständen zur Analytik im Auge verwendet werden kann.

Diese Aufgaben werden gelöst durch die Vorrichtung gemäß Anspruch 1. Insbesondere wird eine Vorrichtung zur gezielten Beleuchtung eines intraokularen Raumes eines menschlichen oder tierischen Auges zur Verfügung gestellt, die sehr flexibel einsetzbar ist und in einfacher Weise verwendet werden kann. Die Vorrichtung kann dem Bedarf angepasst werden. Darüber hinaus bietet die erfindungsgemäße Vorrichtung den Vorteil, dass sie nicht nur auf den jeweiligen Anwendungszweck und für die jeweiligen Erfordernisse einstellbar ist, sondern dass auch für den Nutzer die Möglichkeit besteht, während der Verwendung Anpassungen vorzunehmen. Insbesondere können mit der erfindungsgemäßen Vorrichtung Parameter wie Lichtstärke, Farbe, Intensität, beleuchtete Fläche an den jeweiligen Einsatz angepasst werden. Weiterhin können verschiedene Parameter des Lichtstrahls auch während der Nutzung nach Wunsch verändert werden. Die Vorrichtung umfasst einen Sockel, der an seinem proximalen Ende einen Schaft und an seinem distalen Ende eine Sonde aufweist. Die Sonde ist mit einer Lichtquelle versehen, die wiederum mit einer Energiequelle verbunden ist. Die Lichtquelle weist mindestens eine LED, auf, kann aber auch mehrere LEDs haben, um z.B. einen gewählten Farbwert einzustellen. Wesentlich für die vorliegende Erfindung ist es, dass die Lichtquelle am distalen Ende der Sonde positioniert ist.

Weitere vorteilhafte Ausführungsformen finden sich in den Unteransprüchen.

**Figuren**

Figur 1 zeigt eine Skizze einer Ausführungsform der erfindungsgemäßen Vorrichtung zur gezielten Ausleuchtung des intraokularen Raumes eines menschlichen oder tierischen Auges.
Figur 2 zeigt eine Skizze einer weiteren Ausführungsform der erfindungsgemäßen Vorrichtung zur gezielten Ausleuchtung des intraokularen Raumes eines menschlichen oder tierischen Auges.

Figur 3 zeigt eine Skizze einer weiteren Ausführungsform des erfindungsgemäßen Ophthalmoilluminators.

Figur 4 zeigt eine Skizze des distalen Bereichs des erfindungsgemäßen Ophthalmoilluminators.

Figur 5 zeigt eine Skizze einer für den Einsatz in der erfindungsgemäßen Vorrichtung geeigneten LED.

Figur 6 stellt die Bestrahlungsstärken einer erfindungsgemäßen LED bei verschiedenen Strömen in Abhängigkeit der Wellenlänge des emittierten Lichtes dar.

Figur 7 zeigt Bilder eines Beleuchtungstests mit einem erfindungsgemäßen Ophthalmoilluminator an einem porkinen Auge.

**Bezugszeichenliste**

1     Vorrichtung
2     Sockel
3     Schaft
4     Sonde
5     Lichtquelle
6     Energiequelle
21    Abstandshalter
22    distales Ende des Sockels
23    proximales Ende des Sockels
24    Kanal
30    Halterung
61    Leitung
62    Strombegrenzer
70    LED-Stift
72    Sockel LED-Stift
73    Schaft LED-Stift
74    Sonde LED-Stift
75    Lichtquelle LED-Stift
76    Schalter LED-Stift
80    Trokar
81    Halteteil Trokar
82    Kanüle Trokar

**Definitionen**

Die Bezeichnung "intraokularer Raum", wie sie in der vorliegenden Beschreibung verwendet wird, bezeichnet den gesamten Innenraum des Auges, insbesondere hinter der Sklera und schließt den Augenhintergrund mit ein.

Der Ausdruck "Sockel", wie er hier verwendet wird, betrifft eine Halterung, die auf einer Seite zur Aufnahme einer Sonde und auf der anderen Seite zur Aufnahme eines Schafts geeignet ist.

Der Ausdruck "verbindbar", wie er hier verwendet wird, bedeutet, dass zwei Teile miteinander verbindbar oder verbunden sind. Jede Art von Verbindung ist geeignet, wobei eine dauerhafte ebenso wie eine vorübergehende Fixierung eingeschlossen ist. Verbindbar bedeutet insbesondere, dass ein Teil mit einem anderen zeitweise verbunden werden kann und gegebenenfalls durch ein anderes Teil ausgetauscht werden kann.

Der Ausdruck "Sonde" bezeichnet eine Vorrichtung, die stabförmig ist und innen mindestens einen Kanal aufweist.

Erfindungsgemäß wird eine Lichtquelle mit mindestens einer LED verwendet. Der Begriff "LED" umfasst jede Art von LED, wie sie im Stand der Technik bekannt ist, d.h. insbesondere LED und OLED. Der Begriff "LED" schließt LEDs geringer Größe, z.B. Mikro-LEDs ein.

Der Begriff "Lichtquelle mit mindestens einer LED" bezeichnet solche Lichtquellen, die mindestens eine LED aufweisen, aber auch mehrere LEDs in Kombination aufweisen können. Insbesondere fallen unter diesen Begriff LEDs in allen geeigneten Ausführungsformen sowie Kombinationen von LEDs, die verwendet werden, um eine gewünschte Farbe zu erzeugen, insbesondere RGB-LEDs.

Eine "Energiequelle", wie sie hier verstanden wird, ist jede Vorrichtung, die der erfindungsgemäßen Lichtquelle Energie, insbesondere Strom, zuführen kann, um sie zum Leuchten zu bringen. Beispiele für Energiequellen sind Akkus, Batterien, Kondensatoren etc.

In einer Ausführungsform hat die erfindungsgemäße Vorrichtung die Form eines Stifts und wird in dieser Beschreibung daher als "LED-Stift" bezeichnet.

In der vorliegenden Beschreibung bezieht sich der Begriff "distal" auf von dem Nutzer, z.B. dem Chirurgen, entfernt gelegene Bereiche, während "proximal" sich auf dem Nutzer näherliegende Bereiche der Vorrichtung bezieht.

**Detaillierte Beschreibung der Erfindung**

Die vorliegende Erfindung stellt eine Vorrichtung (1) zur gezielten Beleuchtung eines intraokularen Raumes eines menschlichen oder tierischen Auges bereit, die einen Sockel (2) mit einem proximalen Ende (23) und einem distalen Ende (22), einen Schaft (3) mit einem proximalen Ende und einem distalen Ende, eine Sonde (4) mit einem proximalen Ende und einem distalen Ende, eine Lichtquelle (5) mit mindestens einer LED, eine mit der Lichtquelle (5) verbundene Energiequelle (6) umfasst, wobei der Schaft an seinem distalen Ende mit dem proximalen Ende des Sockels verbindbar ist, die Sonde mit dem distalen Ende des Sockels verbindbar ist und wobei die Lichtquelle (5) am distalen Ende der Sonde (4) positioniert ist. Bei der erfindungsgemäßen Vorrichtung handelt es sich somit um einen Ophthalmoilluminator, der insbesondere zur Ausleuchtung des Auges geeignet ist.

Die erfindungsgemäße Vorrichtung, d.h. der erfindungsgemäße Ophthalmoilluminator, zeichnet sich dadurch aus, dass er sehr flexibel einsetzbar ist, je nach Bedarf angepasst werden kann, das Operationsgebiet gezielt und optimal ausleuchten kann und gleichzeitig das restliche Auge, insbesondere die Netzhaut schützt. Der erfindungsgemäße Ophthalmoilluminator ist daher für die Ophthalmochirurgie sehr gut geeignet, insbesondere für Eingriffe am Augenhintergrund, z.B. an der Netzhaut und für jede Art von Pars-plana-Vitrektomie. Die erfindungsgemäße Vorrichtung zeichnet sich auch dadurch aus, dass sie kompakt ist, dass keine externen Verbindungen mit einer Lichtquelle erforderlich sind, die den Operateur beeinträchtigen könnten. Die Vorrichtung ist sterilisierbar und kann als Wegwerfprodukt eingesetzt werden. Dadurch ist sie überall einsetzbar, auch unter schwierigen Bedingungen. Weiterhin wurde gezeigt, dass mit der erfindungsgemäßen Vorrichtung die in der obengenannten DIN-Norm vorgeschriebenen Grenzwerte eingehalten werden können und dass eine ausreichende Expositionszeit ermöglicht wird.

Der Sockel der erfindungsgemäßen Vorrichtung ist ein Teil, das dazu dient, am distalen Ende eine Sonde und am proximalen Ende einen Schaft zu halten, aufzunehmen oder abzustützen. Der Sockel kann eine Öffnung oder einen Kanal aufweisen, wobei die Öffnung bevorzugt rund bzw. im Wesentlichen rund ist und wobei der Kanal gerade oder gebogen sein kann und der Querschnitt des Kanals bevorzugt rund oder oval ist. Der äußere Teil des Sockels kann jegliche Form haben, bevorzugt ist er rund oder oval. Die äußere Wand kann z.B. gerade, konisch oder doppelkonisch ausgebildet sein. Die Öffnung des Sockels bzw. der Kanal dient dazu, eine oder mehrere Stromleitungen hindurchzuführen. Der Sockel kann eine Aufnahmestelle für den Schaft aufweisen, mit dem Schaft verbunden oder Teil des Schaftes sein. Der Sockel kann dazu dienen, eine Sonde hindurchzuführen oder aber an einem Ende eine Sonde aufzunehmen. Am anderen, dem proximalen Ende, ist ein Schaft anbringbar.

Der Schaft der erfindungsgemäßen Vorrichtung kann jede beliebige Form haben. Er dient dazu, die Vorrichtung zu halten und/oder zu navigieren.

Die Sonde der erfindungsgemäßen Vorrichtung ist stabförmig, wobei der Querschnitt des Stabs jede beliebige Form haben kann. Die Sonde dient dazu die Lichtquelle zu halten oder zu tragen und ggf. eine Verbindung von Lichtquelle zu Energiequelle, z.B. eine Stromleitung aufzunehmen. Die Sonde ist bevorzugt starr.

Eine wesentliche Eigenschaft des erfindungsgemäßen Ophthalmoilluminators ist es, dass der intraokulare Raum gezielt und optimal ausgeleuchtet werden kann. Dies wird zum einen dadurch erreicht, dass die Lichtquelle sich am distalen Ende der Vorrichtung befindet und direkt in das Auge eingestrahlt wird, ohne über einen Lichtleiter geführt zu werden. Dadurch wird erreicht, dass wenige Verluste auftreten und die Effizienz hoch ist. Außerdem kann das Licht direkt über die Bewegung der Sonde dahin gelenkt werden, wo es notwendig ist. Dies vermeidet auch die Notwendigkeit, Licht von einer Lichtquelle zu dem Lichtleiter zu leiten, wozu relativ starre Leitungen notwendig sind, die während eines Eingriffs stören.

Ein weiteres wichtiges Merkmal ist es, dass der von der Lichtquelle emittierte Lichtstrahl vom Benutzer eingestellt bzw. geführt werden kann. Auf diese Weise kann genau der Bereich beleuchtet werden, den der Nutzer, z.B. der Operateur, sehen muss, während andere Bereiche der Netzhaut geschont werden können. Der Lichtstrahl kann durch Bewegung dse Schaftes navigiert werden. Größe, Form und Richtung des Lichtkegels können durch Auswahl und/oder Anpassung der LED und/oder der Sonde und durch Navigierung verändert werden.

Der Abstrahlwinkel einer LED kann mit der Formel

$$\alpha = 2 * \left( \arctan\left(\frac{d}{l * 2}\right) \right)$$

berechnet werden, wobei

α = Abstrahlwinkel

d = Durchmesser des zu beleuchtenden Objektes/Raumes

l = Abstand der Lichtquelle zur beleuchtenden Fläche

arctan = Umkehrfunktion der Tangensfunktion

Entsprechend kann eine geeignete LED für den jeweiligen Zweck ausgewählt werden. Der Abstrahlwinkel kann verändert werden, z.B. durch einen Reflektor begrenzt werden oder der Lichtstrahl kann durch eine Linse gestreut werden. Die Richtung des Lichtsstrahls/Lichtkegels kann verändert werden, indem der Bereich der Lichtquelle, der den Lichtkegel aussendet, entsprechend ausgebildet wird. Diese Anpassungen sind dem Fachmann bekannt. Der Abstrahlwinkel (Öffnungswinkel) α der Lichtquelle kann z.B. auch durch entsprechende Verkapselung der LED eingestellt werden.

Eine weitere Möglichkeit, den Lichteinfall zu beeinflussen, besteht darin, einen Teil der Oberfläche der LED so zu beschichten, dass kein oder wenig Licht austritt, sodass die Hauptmenge an Licht über einen gewünschten Bereich austreten kann. Damit kann dann bei der Beleuchtung noch gezielter die gewünschte Stelle angestrahlt werden, wobei empfindlichere Stellen von der Beleuchtung dann ausgenommen werden.

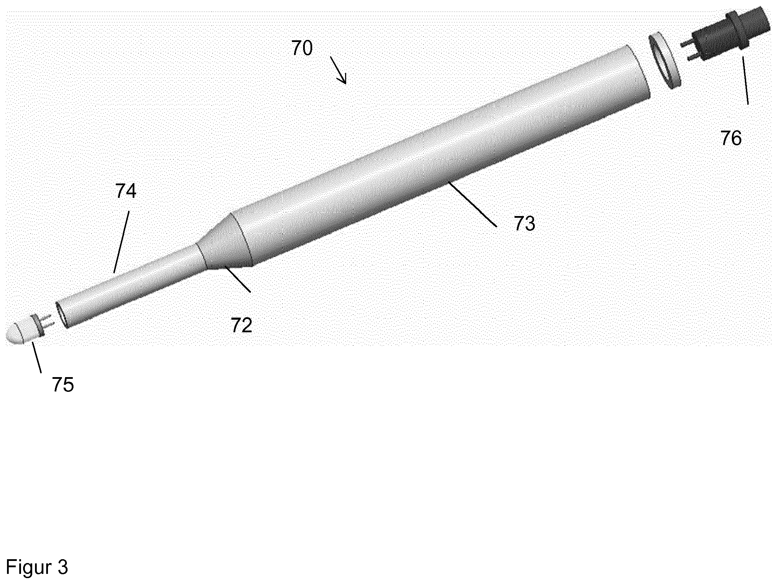

In einer weiteren Ausführungsform wird der Lichtkegel dadurch beeinflusst, dass die Lichtquelle in einem Winkel ß an der Sonde angebracht wird. Der Winkel ß bezieht sich dabei auf den Winkel, der zwischen der Längsachse (A) der Sonde und der Unterseite der Lichtquelle gebildet wird. So kann z.B. die Lichtquelle (5) in einem Winkel ß von 5° *bis 90°*, bevorzugt von 10° bis 80°, bevorzugt von 20° bis 70°, bevorzugt von 30° bis 60°, bevorzugt von 40° bis 50°, bevorzugt von 45° zur Längsachse (A) des Schaftes angeordnet sein, wie es beispielhaft in Figur 3 b und 3 c gezeigt ist.

Der von der Lichtquelle emittierte Lichtkegel kann über den Schaft der Vorrichtung manuell durch Drehen oder Kippen gezielt navigiert werden. Eine Veränderung des Lichtkegels durch Drehen erfolgt insbesondere dann, wenn die Lichtquelle nicht direkt vorne auf der Sonde aufsitzt, sondern in einem Winkel dazu angebracht ist. Die Veränderung kann auch durch Kippen der Vorrichtung erfolgen. Hierzu ist in einer bevorzugten Ausführungsform der Schaft so ausgebildet, dass er leicht vom Operateur bedient werden kann. Beispielsweise kann der Schaft Längsrippen aufweisen, um seine Griffigkeit zu erhöhen.

Die einfache Bedienbarkeit, d.h. Drehen oder Kippen, wird dadurch erzielt, dass die erfindungsgemäße Vorrichtung über den Sockel in einem Gewebe "verankert" oder gehalten werden kann. Mit anderen Worten kann der Sockel in Gewebe gesteckt werden, insbesondere das Pars-plana-Gewebe, das den Augapfel umgibt. In dieser Ausführungsform kann der Sockel beispielsweise einfach oder doppelt konisch geformt sein, um das "Verankern" zu erleichtern. Unter Verankern wird hierbei verstanden, dass der Sockel in einen Gewebebereich geschoben wird und dort an Ort und Stelle verbleibt. Die Sonde der erfindungsgemäßen Vorrichtung kann auch in einen bereits in das Gewebe insertierten Trokar geführt werden, wobei der Trokar dann für die Verankerung sorgt.

Aufgrund der im Winkel ß angeordneten Lichtquelle kann ein Operationsgebiet an Stellen optimal und gewebeschonend ausgeleuchtet werden, die mit bekannten Geräten nicht erreicht werden, z.B. Bereiche in der Nähe der Insertionsstelle oder an der Peripherie der Retina. Andererseits kann durch einen am Sockel vorgesehenen Abstandshalter verhindert werden, dass die Sonde mit der LED zu nahe an die empfindliche Netzhaut kommt.

So ist es möglich, den Lichtkegel im Auge extrem zielgerichtet auf die zu beleuchtende Stelle zu richten. Auch kann gezielt die Beleuchtung empfindlicher Stellen, wie beispielsweise die Makula, von der Beleuchtung ausgenommen werden Gleichzeitig wird durch die zielgerichtete Navigation und eingeschränkte Größe des Lichtkegels vermieden, dass weiteres Gewebe unnötig der Licht- und Wärmestrahlung ausgesetzt wird.

Die LED kann mit jedem für diese Art von Lichtquelle geeigneten Material, insbesondere transparentem Material verkapselt sein. Geeignet ist beispielsweise transparentes Glas, Keramik oder ein Polymer wie Epoxidharz, Acrylharz, Plexiglas, Polymethylmethacrylat, Polyurethan oder Silikon. Die Wahl des Materials bestimmt die optische Qualität, die in der Regel bei härteren Materialien wie Glas höher ist, und die Gewebeverträglichkeit, die eher bei weicheren Materialien wir Silikon höher ist. Es kann auch ein Material verwendet werden, dass als Diffusor wirkt oder das Licht durch Beugung in gewünschter Weise verändert.

Die Lichtquelle kann eine plane, konkave oder konvexe Oberfläche mit einem runden, abgerundeten oder mehreckigen Querschnitt haben. Vorteilhafterweise sind die Kanten der Lichtquelle, bzw. der Verkapselung der Lichtquelle abgerundet bzw. abgekantet, um Absplitterungen während der Verwendung des Ophthalmoilluminators am Auge zu vermeiden.

Sowohl Kapselmaterial als auch Kapselform können gezielt gewählt werden, um die Lichtführung, Abstrahlrichtung und Lichtqualität der Lichtquelle zu beeinflussen.

Die erfindungsgemäße Vorrichtung kann weiterhin eine Steuereinrichtung umfassen, über die die Farbtemperatur und/oder Wellenlänge und/oder Helligkeit und/oder Intensität des Lichts einstellbar ist. Die Steuereinrichtung kann in üblicher Art und Weise ausgebildet sein. Es kann sich z.B. um eine drahtlose Schnittstelle handeln, die über eine Fernbedienung gesteuert werden kann. Jede mögliche Art der Steuerung ist hier möglich, z.B. auch eine Steuerung über Sprache oder Bewegung.

Um die Farbtemperatur des Lichts einzustellen, kann entweder gezielt eine LED in der gewünschten Farbtemperatur oder eine Kombination von zwei oder mehr LEDs gewählt werden, die dann die gewünschte Farbe bilden können. Ein Beispiel ist eine als RGB LED bezeichnete Lichtquelle, die aus drei hintereinandergeschalteten LEDs besteht, aus denen sehr viele gewünschte Farben gemischt werden können. Eine Veränderung der Farbtemperatur kann vorteilhaft sein, z.B. um spezifische Gewebestrukturen unter speziellem Licht besser erkennen zu können, um Operationsgeräte unter speziellem Licht besser erkennen und einsetzen zu können, um spezifisch für die Färbung von Strukturen eingesetzte Farbstoffe besser erkennen zu können, oder um die Farbtemperatur speziell auf einen Nutzer einstellen zu können. So kann z.B. die Wellenlänge vor und/oder während einer Operation auf verwendete Farbstoffe, Farbpigmente bzw. nanoskalige fluoreszierende Partikel oder Flüssigkeiten abgestimmt werden.

Weiterhin kann die Helligkeit der Lichtquelle über eine gezielte Schaltung mit verschiedenen Strömen eingestellt werden.

Die Steuereinrichtung, über die die Farbtemperatur des Lichts und/oder die Helligkeit des Lichts einstellbar ist, kann als Schalter mit verschiedenen Schaltstufen ausgeführt sein. In einer anderen Ausführungsform erfolgt die Schaltung elektronisch, z.B. über eine Sprachsteuerungseinheit oder eine Fernbedienung.

Die Einstellung der Parameter Austrittswinkel des Lichts, Abstrahlwinkel des Lichts, Farbtemperatur und Helligkeit ermöglichen die bestmögliche Ausleuchtung des Operationsgebietes im Auge bei gleichzeitiger bestmöglicher Schonung des betroffenen und des angrenzenden Gewebes, sowie bestmöglichem Makulaschutz.

Aus den verschiedenen Teilen der erfindungsgemäßen Vorrichtung kann flexibel eine für den jeweiligen Zweck besonders gut geeignete Vorrichtung gestaltet werden. Wesentliche Teile hierzu sind die Sonde, der Sockel, der Schaft und die Lichtquelle. Alle Teile der Vorrichtung können aus an sich für diese Anwendung üblichen Materialien hergestellt werden. Das jeweilige Material sollte biokompatibel und sterilisierbar sein.

Der Sockel dient dazu, die Vorrichtung bei intraokularer Verwendung im Gewebe zu halten und/oder zu verankern und die Sonde auf der distalen Seite und den Schaft auf der proximalen Seite zu halten. Der Sockel ist hierzu als Teil mit einer Öffnung ausgebildet. Der Sockel kann aus üblichen biokompatiblen und bevorzugt auch elektrisch isolierenden Materialien hergestellt sein. Beispielsweise kann der Sockel aus gewebeverträglichem elastomeren Material, z.B. elastomerem Silicon, elastomeren Polymeren etc. hergestellt sein. Durch eine gewisse Elastizität kann sich der Sockel dann der Umgebung anpassen. Allerdings darf das Material nicht zu flexibel sein, da der Durchgang, d.h. die Öffnung, zugänglich sein sollte. Bei geeigneter Formgebung ist ebenfalls auch ein Duroplast als Material für den Sockel geeignet. Darüberhinaus kann der Sockel dazu dienen, die Vorrichtung an einer gewünschten Stelle im Gewebe, z.B. im Pars-plana-Bereich und im geeigneten Abstand zu halten.

Auf der distalen Seite des Sockels kann eine Sonde positioniert werden. Die Sonde dient dazu, an ihrer Vorderseite die Lichtquelle zu tragen und in ihrem Inneren die Verbindung von der Lichtquelle zur Energiequelle zuzulassen. Hierzu werden in der Regel Verbindungen, z.B. Drähte, verwendet, die die Lichtquelle mit der proximal des Sockels gelegenen Energiequelle verbinden. Die Sonde soll möglichst dünn sein, um möglichst wenig Gewebe zu schädigen. Das Material der Sonde sollte elektrisch isolierend sein.

Bevorzugt liegt die Dicke der Sonde im Bereich von 20 bis 30 gauge, besonders bevorzugt 23 bis 28 gauge.

Ein weiterer wesentlicher Teil der erfindungsgemäßen Vorrichtung ist ein Schaft, der an der proximalen Seite des Sockels positioniert ist. Der Schaft kann entweder fest mit dem Sockel verbunden sein oder aber der Sockel kann so ausgebildet sein, dass er den Schaft aufnehmen und halten kann. Der Schaft dient dazu, die Vorrichtung zu bedienen, d.h. zu positionieren, zu drehen und zu kippen. In einer Ausführungsform ist der Schaft daher so ausgebildet, dass er Griffmulden, Griffrippen, Griffflächen oder Ähnliches aufweist, damit er leicht vom Nutzer gedreht und gehalten werden kann. Der Schaft kann aus jedem möglichen Material hergestellt sein, solange das Material biokompatibel ist.

Die erfindungsgemäße Vorrichtung kann in einer Ausführungsform zum leichteren Einsetzen zusätzlich zu dem Schaft noch einen Handgriff aufweisen, der abnehmbar an dem Schaft oder an dem Sockel positionierbar ist. Nach dem Einsetzen der Vorrichtung kann der Handgriff abgenommen werden. Es ist auch möglich, dass an dem Sockel zuerst ein Handgriff positioniert ist zum Einsetzen der Vorrichtung und nach dem Einsetzen der Handgriff dann durch den Schaft ausgetauscht wird. Der Handgriff ist in der Regel größer als der Schaft und könnte während eines Eingriffs behindern, so dass es vorteilhaft ist, ihn nach dem Einsetzen zu entfernen.

Die Lichtquelle (5) ist mit einer Energiequelle elektrisch verbunden, wobei die Lichtquelle distal an einer Sonde positioniert ist, während die Energiequelle bevorzugt außerhalb des intraokularen Raums an der Vorrichtung angeordnet ist, z.B. im proximalen Bereich des Schaftes. Die Verbindung von Lichtquelle und Energiequelle kann konaktlos oder über elektrische Leitung erfolgen. In einer Ausführungsform ist die Lichtquelle über einen Schalter mit einer Energiequelle (6) verbunden. Die Energiequelle (6) kann eine oder mehrere Batterien, einen oder mehrere Akkus, und/oder einen oder mehrere Kondensatoren umfassen. In einer bevorzugten Ausführungsform weist die Energiequelle mindestens eine Batterie auf. Geeignet sind Batterien wie sie für solche Geräte verwendet werden. Bevorzugt werden hierbei Lithiumionen-Batterien verwendet, da diese einen sehr kontinuierlichen Strom liefern und somit die Leuchtintensität der Lichtquelle ziemlich konstant bleibt. Eine konstante Lichtintensität trägt zu den vorteilhaften Eigenschaften des erfindungsgemäßen Ophthalmoilluminators bei.

Um die Stabilität des ausgestrahlten Lichts weiter zu verbessern und die Lichtintensität konstant zu halten, kann in einer weiteren bevorzugten Ausführungsform zusätzlich ein Strombegrenzer oder LED-Treiber verwendet werden.

Bevorzugt befindet sich die Energiequelle in einem dafür vorgesehenen Gehäuse. Das Gehäuse wird vorzugsweise nahe des proximalen Bereichs des Schaftes fixiert oder in den Schaft integriert, um eine möglichst kompakte Vorrichtung zur Verfügung zu stellen. Möglich ist auch die Verwendung von mindestens einem Akku, der berührungslos oder durch Verbindung mit einem Ladegerät geladen werden kann. So kann das Gehäuse, das die Energiequelle enthält und mit der Lichtquelle verbunden ist, während eines Eingriffs beispielsweise in der Nähe der Vorrichtung, z.B. auf die Stirn des Patienten, aufgeklebt werden.

Die Verwendung von Batterien oder Akkus als Energiequelle ermöglicht den flexiblen Einsatz des erfindungsgemäßen Ophthalmoilluminators.

In einer Ausführungsform der erfindungsgemäßen Vorrichtung ist die Sonde an dem Sockel befestigt und wird über eine Halterung und/oder den Schaft durch das Gewebe geführt, z.B. im Pars-plana-Bereich. In dieser Ausführungsform wird zuerst eine Öffnung im Gewebe erzeugt, z.B. durch eine Inzision, durch die dann die Sonde mit dem Sockel geschoben werden kann.

In einer weiteren Ausführungsform wird zuerst mit Hilfe eines üblichen Punktionssinstruments, z.B. ein Trokar in das Gewebe gestochen. Anschließend wird dann die erfindungsgemäße Sonde durch den Trokar, in den Intraokularraum geschoben. Für diese Ausführungsform muss die Sonde mit Lichtquelle einen kleineren Durchmesser haben als der Trokar, um durchgeschoben werden zu können. In dieser Ausführungsform ist bevorzugt die Sonde (4) mit reduziertem Außendurchmesser ausgestaltet, z.B. 18 bis 30 gauge, bevorzugt 23 bis 28 gauge.

Die oben beschriebenen Ausführungsformen haben viele Vorteile gegenüber bekannten Vorrichtungen: sind einfach in der Anwendung, sehr flexibel und können an viele verschiedene Gegebenheiten angepasst werden. Sie können in den Intraokularraum mit minimal invasiver Technik eingeführt werden, liefern eine optimale Ausleuchtung, die außerdem noch während des Eingriffs bzgl. verschiedener Paramerter angepasst werden kann.

In einer weiteren Ausführungsform des erfindungsgemäßen Ophthalmoilluminators befinden sich alle Teile, d.h. Sockel, Schaft, Sonde, Lichtquelle, Energiequelle und Gehäuse bereits fertig montiert kompakt in einem Gehäuse. Diese Ausführungsform ist geeignet für transsklerale Illumination. Die erfindungsgemäße Vorrichtung kann dabei ausgebildet sein wie ein Stift, an dessen Spitze sich eine Lichtquelle befindet. Das Gehäuse kann über die gesamte Länge im wesentlichen den gleichen Außendurchmesser aufweisen. Diese Ausführungsform kann insbesondere dazu verwendet werden, um den intraokularen Raum transdermal zu beleuchten. Dazu kann die Vorrichtung in der Bindehauttasche geführt werden und den intraokularen Raum über die Sklera beleuchten. Der Stift wird mit der Hand geführt, d.h. der Nutzer kann das Licht genau dahin lenken, wo er es braucht. Das Licht fällt durch die Sklera. Dieser Stift dient für Diagnostik, zur Beleuchtung und als Deller. Bei dieser Ausführungsform wird die Netzhaut von außen beleuchtet und Risse und Löcher werden für den Nutzer gut sichtbar. Diese Ausführungsform ist daher gut geeignet für die nicht-invasive Untersuchung. Ein weiterer Vorteil dieser Vorrichtung ist es, dass keine nach außen gehenden Drähte, Verbindungen oder Halterungen notwendig sind. Der Stift kann als Wegwerfprodukt hergestellt werden. Er wird der Verpackung entnommen, ist steril, wird verwendet und dann weggeworfen. Er ist daher gut geeignet für den Einsatz im Feld, in schwierigen Gebieten, im Lazarett usw.

In einer Ausführungsform dieser stiftförmigen Vorrichtung können mehrere LEDs, z.B. RGB-LEDs verwendet werden, da hier genug Platz dafür ist. Dann kann die Farbe des Lichtes jeweils vom Nutzer nach Wunsch eingestellt werden.

In einer anderen Ausführungsform ist ein proximaler Bereich (11) des Schaftes als Handstück (42) zur Navigation des Lichtkegels ausgestaltet. Das Handstück weist bevorzugt einen größeren Außendurchmesser als der Rest des Schaftes auf. In einer weiteren Ausführungsform ist die Oberfläche des Handstückes und/oder des Schaftes modifiziert, um eine bessere Griffigkeit zur Navigation des Schaftes und damit des Lichtkegels zu erreichen. Alternativ kann ein kleiner Griff in Form eines senkrecht zum Schaft angebrachten Stabes oder einer anderweitigen Ausstülpung am Handstück angebracht werden, um die Feinnavigation zu erleichtern.

Ferner ist in einer weiteren Ausführungsform der erfindungsgemäßen Vorrichtung im proximalen Bereich des Schaftes (11) eine Halterung (42) angebracht. Diese Halterung dient der besseren Handhabbarkeit z.B. bei der Insertion des erfindungsgemäßen Ophthalmoilluminators in eine am Pars plana eingebrachte Insertion. Die Halterung ist so am Schaft angebracht, dass sie de-arretiert und entfernt werden kann, sobald diese nicht mehr benötigt wird. Es bleibt dann der Schaft oder das Handstück aus der alternativen Ausführungsform zurück und ermöglicht eine Feinnavigation des Schaftes, bzw. des Lichtkegels.

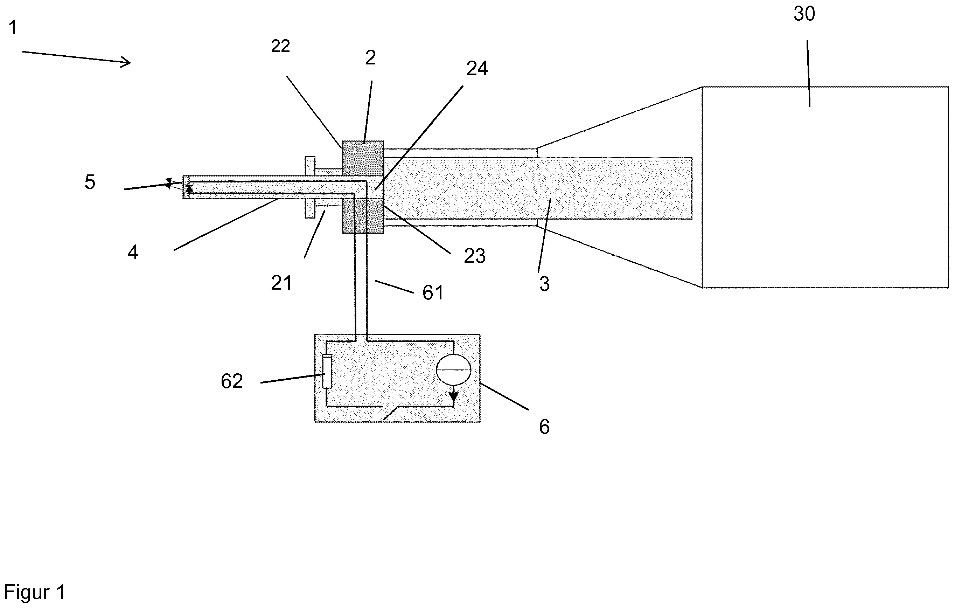

Fig. 1 zeigt eine Ausführungsform der erfindungsgemäßen Vorrichtung zur Beleuchtung eines intraokularen Raumes eines menschlichen oder tierischen Auges. Sockel 2 trägt am distalen Ende 22 eine Sonde 4. An dem Sockel befindet sich außerdem ein Abstandshalter 21, der dafür sorgt, dass die Sonde nicht zu weit in den intraokularen Raum hineingeschoben werden kann, um dadurch eine zu starke Strahlenbelastung bzw. Verletzungen durch die Sonde zu verhindern. Am distalen Ende der Sonde 4 befindet sich als Lichtquelle eine LED 5. Die Lichtquelle ist mit einer außerhalb der Vorrichtung angeordneten Energiequelle 6 über eine Leitung 61 verbunden. Die Leitung führt von der LED 6 durch die Sonde 4 und einen Kanal 24 in dem Sockel 2 zu dem Batteriegehäuse 6, in dem sich ggf. eine oder mehrere Batterien (nicht gezeigt) als Energiequelle befinden. An der proximalen Seite 23 des Sockels 2 befindet sich ein Schaft 3, der zur Navigation des Lichtkegels vorgesehen ist, wenn die Vorrichtung zur Beleuchtung in Betrieb genommen wird. Auf den Schaft 3 ist eine Halterung 30 aufgesteckt, die zur Applikation der Vorrichtung dient und, sobald die Vorrichtung im Gewebe verankert ist, abgenommen werden kann.

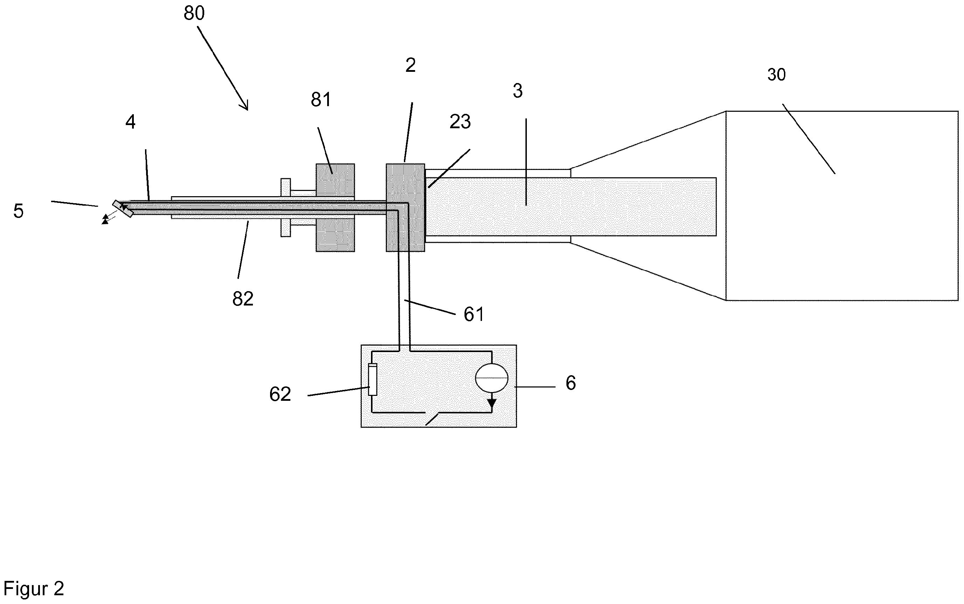

Eine weitere Ausführungsform der erfindungsgemäßen Vorrichtung ist in Fig. 2 dargestellt. Die in Fig. 2 dargestellte Vorrichtung zur gezielten Beleuchtung eines intraokularen Raumes eines menschlichen oder tierischen Auges weist einen Sockel 2 auf, der am proximalen Ende 23 einen Schaft 3 trägt, der zur Navigation des Lichtkegels geeignet ist. Auf den Schaft 3 aufgesteckt ist eine Halterung 30, die zur Applikation der Vorrichtung dient und abgenommen werden kann, sobald die Vorrichtung im Gewebe verankert ist. Am distalen Ende 21 trägt der Sockel 2 eine Sonde 4, an deren distalem Ende eine LED 5 so positioniert ist, dass sie mit der Achse der Vorrichtung einen Winkel von 45° bildet. Von der LED 5 führen Drähte 61 durch einen Kanal (nicht gezeigt) in der Sonde 4 hindurch in den Sockel 2 und über einen im Winkel angeordneten im Sockel vorgesehenen Kanal aus der Vorrichtung hinaus hin zu einem Batteriegehäuse 6, das eine oder mehrere Batterien (nicht gezeigt) aufweist. Dadurch ist die LED 5 mit einer Stromquelle, dem Batteriegehäuse 6 verbunden. Das Batteriegehäuse 6 weist außerdem einen Strombegrenzer 62 auf. Die Sonde 4 führt in der Ausführungsform der Fig. 2 durch einen Trokar 80 hindurch, der im Gewebe verankert ist. Der Trokar 80 besteht aus einem Halteteil 81 und einer Kanüle 82. Die Kanüle 82 dient dazu, das Gewebe zu durchstechen und die Sonde des erfindungsgemäßen Ophthalmoilluminators zu führen und zu halten. Bei Verwendung wird die Sonde 4 der erfindungsgemäßen Vorrichtung durch den in dem Trokar 80 und die Kanüle 82 gebildeten Kanal in den Intraokularraum geführt, um intraokular zu beleuchten. Wenn die Beleuchtung nicht mehr notwendig ist, kann die Vorrichtung wieder herausgezogen werden und andere Instrumente können durch den Kanal 82 des Trokars 80 eingeführt werden. Sobald wieder Beleuchtung notwendig ist, kann wiederum die Sonde eingeführt werden. Diese Ausführungsform ist sehr schonend und flexibel, da ein insertierter Trokar für verschiedene Zwecke genutzt werden kann, der Trokar als Anschlag dient, damit die Sonde nicht zu weit in den Okularraum geführt werden kann, und nur eine Inzision im Gewebe notwendig ist für Beleuchtung und Instrumente. Diese vorteilhafte Ausführungsform ermöglicht es, bei der Ophthalmoillumination am Pars plana, zunächst nur den Instrumentenkanal in die Inzision am Auge einzubringen und dann im zweiten Schritt den Ophthalmoilluminator durch den Instrumentenkanal schonend ins Auge einzubringen und auch frei zu drehen, ohne weitere Reibung an der Sklera.

Fig. 3 zeigt eine weitere Ausführungsform der erfindungsgemäßen Vorrichtung, die alle erfindungswesentlichen Komponenten in einem stiftförmigen Gehäuse enthält - einen LED-Stift 70. Diese sehr kompakte Ausführungsform, die keinerlei externe Quellen für Energie oder Licht benötigt, ist überall einsatzbereit. Fig. 3 zeigt das Gehäuse 70 mit einer Sonde 72, an deren distalem Ende eine Lichtquelle 75 (LED) aufsteckbar ist. Ein konusförmiger Sockel 73 befindet sich zwischen Sonde 74 und Schaft 73. Am proximalen Ende des Schafts 73 ist ein Schalter aufsteckbar, mit dem die Lichtquelle an- und ausgeschaltet werden kann. In dem Gehäuse befindet sich eine Energiequelle (nicht gezeigt), die über eine Leitung mit der Lichtquelle verbunden ist (ebenfalls nicht gezeigt). Die LED des Stifts kann in der Bindehauttasche geführt werden und beleuchtet die Netzhaut von hinten, sodass der Benutzer Risse und Löcher gut sehen kann.

Alle Ausführungsformen wurden im Labor getestet und erfüllten die geforderten Grenzwerte und erlaubten eine Ausleuchtung des intraokularen Raums im gewünschten Umfang.

Die erfindungsgemäße Vorrichtung eignet sich zur Verwendung in chirurgischen und diagnostischen Verfahren am Auge, insbesondere zur minimalinvasiven, intraokularen Beleuchtung des intraokularen Raumes des menschlichen oder tierischen Auges.

Die erfindungsgemäße Vorrichtung eignet sich auch zur Verwendung in chirurgischen und diagnostischen Verfahren am Auge, wenn eine nicht-invasive, transsklerale Beleuchtung des intraokularen Raumes des menschlichen oder tierischen Auges gewünscht ist.

Für diese Anwendung sind insbesondere die Ausführungsformen der Vorrichtung geeignet, welche eine Lichtquelle mit einer abgerundeten Spitze haben. Diese kann dann auch verwendet werden, um bei der transskleralen Illumination gezielten Druck auf die Netzhaut auszuüben, um einerseits die Sicht zu verbessern und andererseits die Netzhaut beispielsweise bei einer Laserbehandlung zu fixieren.

Vorteil aller Vorrichtungen ist es, dass keine nach außen führenden Verbindungen zu Lichtquellen erforderlich sind. Die Vorrichtung kann als Stift ausgeführt sein, welcher vorzugsweise als steriles Einmalprodukt ausgestaltet ist. Die Vorrichtung kann daher überall infrastrukturunabhängig verwendet werden und ist einfach in der Handhabung.

Die Erfindung wird auch durch die folgenden Beispiele, Vorrichtungen und Verwendungen und Verfahren, sowie Bestandteile derselben beschrieben und erläutert, ohne sie darauf zu beschränken.

**Beispiele**

**Beispiel 1: Stromversorgung durch ein Batterie-Reservoir**

- Die Stromversorgung wird durch ein Batterie-Reservoir ermöglicht. Diese kann mit mehreren Funktionen ausgestattet werden:

  ◦ Einfacher Schalter
  ◦ Schaltung mit verschiedenen Strömen z.B.:

    ▪ I = 5 [mA]
    ▪ I = 10 [mA]
    ▪ I = 15 [mA]
    ▪ I = 20 [mA]
    ▪ Steuerung durch Mikrokontroller

  o Steuerung durch Fernbedienung

**Beispiel 2: Leistungsdaten einer geeigneten LED:**

Eine für den Einsatz in der erfindungsgemäßen Vorrichtung geeignete LED hat folgende technische Daten:

| | |
|---|---|
| Leuchtfarbe: | Weiß |
| Farbkoordinaten: | x=0,31 y=0,31 |
| Lichtleistung: | typ. 98 mcd |
| Farbtemperatur: | typ. 5500 K |
| Spannung: | typ. 2,9 Volt |
| Strom: | max. 10 mA |
| Abstrahlwinkel: | 135° |
| Arbeitstemperatur: | - 40°C bis + 85°C |
| Löttemperatur: | 5 Sekunden 260°C |
| Maße: | 0,65 x 0,35 x 0,2 mm |

**Beispiel 3: Konstruktionsdaten einer geeigneten LED**

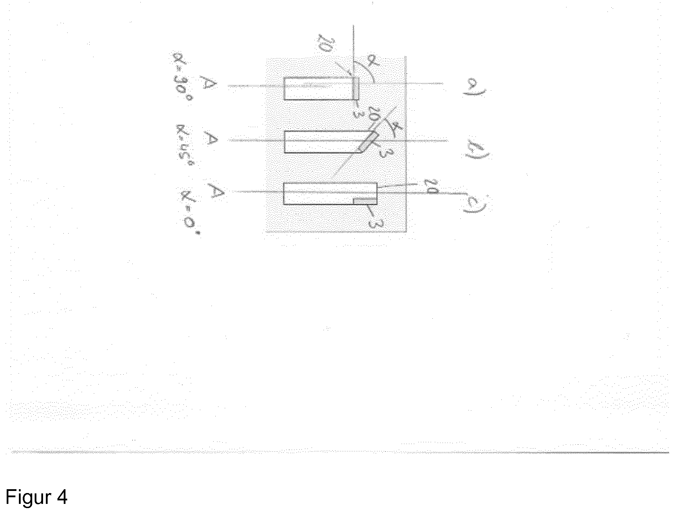

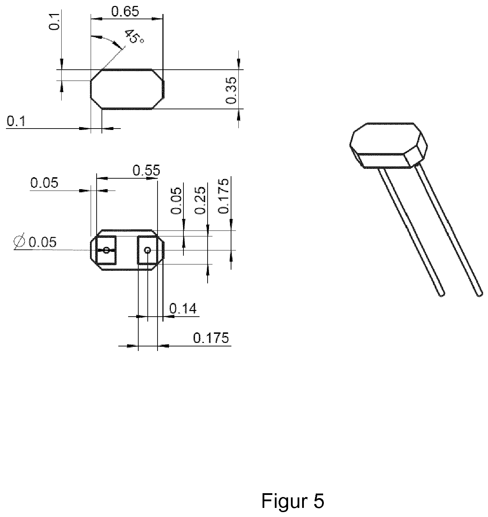

Figur 5 zeigt ein Beispiel für eine für den Einsatz in der erfindungsgemäßen Vorrichtung geeignete LED. Die in Figur 4 dargestellte LED hat eine plane Oberfläche mit einem mehreckigen, hier achteckigen Querschnitt und abgetrennte oder abgerundete Kanten, um eine Absplitterung der Kanten während der Verwendung am Auge zu vermeiden.

**Beispiel 4: Bestrahlungsstärke der LED:**

Die Bestrahlungsstärke der gesamten LED wurde mittels Ulbrichtkugel und gemittelten Werten einer Kalibrationslampe bei den Strömen I = 5, 10, 15, 20 [mA] bestimmt.
Tabelle 1 stellt die so ermittelten Bestrahlungsstärken dar, die in einem Abstand von 13 mm gemessen wurden:.

**Tabelle 1:**

| Stromstärke | Bestrahlungsstärke: [300 - 850 nm] | |
|---|---|---|
| 5 [mA] | $[\mu W/cm^2]$ | 354,5 |
| 10 [mA] | $[\mu W/cm^2]$ | 668,3 |
| 15 [mA] | $[\mu W/cm^2]$ | 911,6 |
| 20 [mA] | $[\mu W/cm^2]$ | 1112,9 |

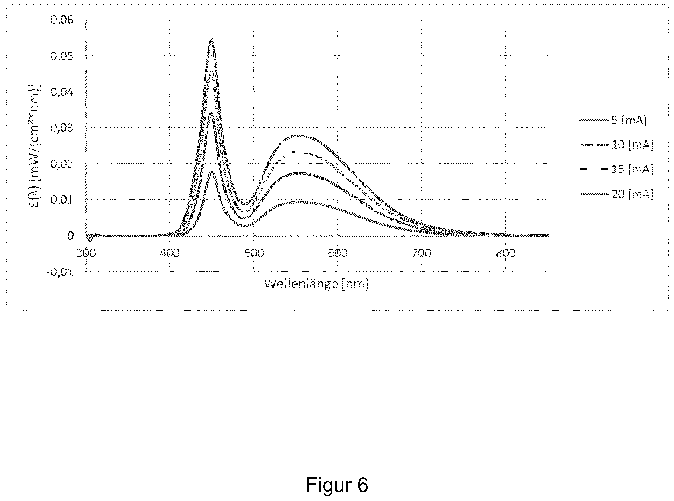

Figur 6 stellt die bei verschiedenen Strömen gemessenen Bestrahlungsstärken in Abhängigkeit der Wellenlänge des emittierten Lichtes dar.

**Beispiel 5: Bewertung nach ISO EN DIN15000-2.2104:**

Die LED wurde auf ihre photochemische Gefährdung hin untersucht und bewertet. Bestimmt wurden die gemessenen Werte mittels Ulbrichtkugel und gemittelten Werten einer Kalibrationslampe. Die gemessenen Werte wurden mittels einer 1 mm Blende erstellt, die restlichen Bewertungen wurden rechnerisch bestimmt.

Tabelle 2 stellt die so ermittelten Werte dar. Die kursiv gedruckten Werte stellen den "worst-case"-Fall dar. Es ergeben sich hier mögliche Zeiten von zwischen 48 min und 140 min, welche wiederum realistisch für eine erfolgreich klinische Anwendung sind. Die ermittelten Werte zeigen, dass mit der hier gewählten LED bei der Verwendung in der erfindungsgemäßen Vorrichtung keine Gefährdung des Augengewebes zu erwarten ist.

**Tabelle 2:**

Details der Messung:

| Durchmesser Blende: | Abstand zur Blende: | Fläche der Messung: |
| --- | --- | --- |
| [mm] | [mm] | [cm$^2$] |
| 1,00 | 13,0 | 7,8540E-03 |
| 0,18 | 13,0 | 2,5447E-04 |
| 0,03 | 13,0 | 7,0686E-06 |

Photochemische Gefährdung:

Berechnung des Bestrahlungsstärke $E_{A-R}$ nach DIN EN ISO 15004-2:2014

| Grenzwert: | Fläche der Messung: | I = 5 [mA] | I = 10 [mA] | I = 15 [mA] | I = 20 [mA] |
| --- | --- | --- | --- | --- | --- |
| [$\mu$W/cm$^2$] | [cm$^2$] | [$\mu$W/cm$^2$] | [$\mu$W/cm$^2$] | [$\mu$W/cm$^2$] | [$\mu$W/cm$^2$] |
| 440,000 | | | | | |
| | 1,00 | 1,1768 | 1,9498 | 2,729 | 3,5271 |
| | 0,18 | 0,0381 | 0,0632 | 0,088 | 0,1143 |
| | 0,03 | 0,0011 | 0,0018 | 0,002 | 0,0032 |

Die Tabelle zeigt, dass für alle gemessenen Proben die Grenzwerte bzgl. der photochemischen Gefährdung eingehalten wurden..

Bestimmung der maximalen Emissionzeit $t_{max}$ durch die ermittelte Bestrahlungsstärke $E_{A-R}$ und der zulässigen gesamten Energiemänge pro Fläche von 10 J/cm$^2$, nach DIN EN ISO 15004-2:2014:

| Grenzwert : | Fläche der Messung: | I = 5 [mA] | I = 10 [mA] | I = 15 [mA] | I = 20 [mA] |
| --- | --- | --- | --- | --- | --- |
| [J/cm$^2$] | [cm$^2$] | [h] | [h] | [h] | [h] |
| 10 | | | | | |
| | 1,00 | 2,4 | 1,4 | 1,1 | 0,8 |
| | 0,18 | 72,9 | 44,0 | 31,4 | 24,3 |
| | 0,03 | 2622,8 | 1583,0 | 1131,2 | 875,1 |

Die erhaltenen Werte zeigen, dass für die erfindungsgemäßen Vorrichtungen Expositionszeiten von weit mehr als 30 Minuten möglich sind. Dies lässt ihren breiten Einsatz in der Ophthalmochirurgie und -diagnostik zu.

**Beispiel 6: Test der Mikro-LED-Illumination an einem porkinen Auge**

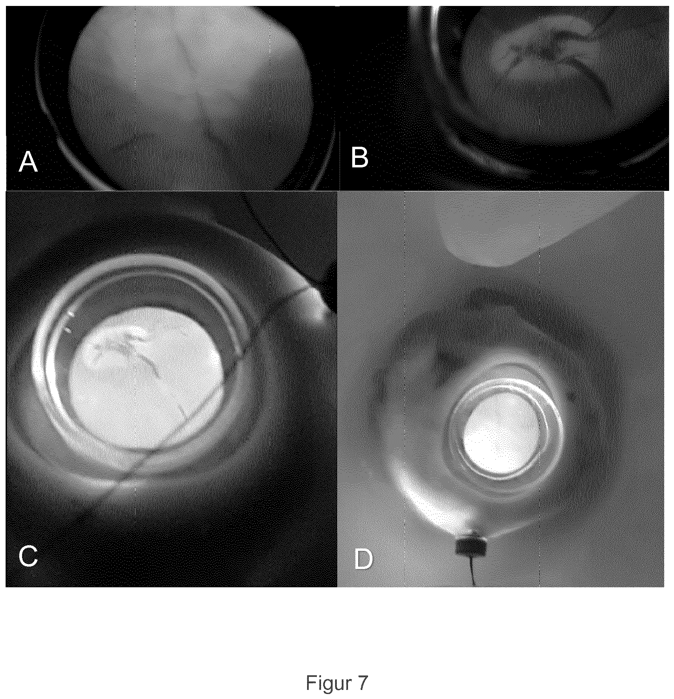

Figur 7 zeigt Bilder eines Beleuchtungstests mit einem erfindungsgemäßen Ophthalmoilluminator an einem prokinen Auge. A und B zeigen Bilder die mit einer Okularkamera mit sehr niedriger Belichtungszeit mit einem Zeiss-Operationsmikroskop aufgenommen wurden. C und D wurden mit einer Handykamera aufgenommen und spiegeln sehr gut das Helligkeitsempfinden wider. In D zeigte der Kegel der LED nicht in Richtung N. opticus, wie an der hellen Stelle an der Seite des Auges zu erkennen ist.

**Patentansprüche**

1. Vorrichtung (1) zur gezielten Beleuchtung eines intraokularen Raumes eines menschlichen oder tierischen Auges umfassend

   a) einen Sockel (2) mit einem proximalen Ende und einem distalen Ende,
   b) einen Schaft (3), mit einem proximalen Ende und einem distalen Ende,
   c) eine Sonde (4), mit einem proximalen Ende und einem distalen Ende,
   d) eine Lichtquelle (5) mit mindestens einer LED, und
   e) eine mit der Lichtquelle (5) verbundene Energiequelle (6),

   wobei der Schaft an seinem distalen Ende mit dem proximalen Ende des Sockels verbindbar ist, die Sonde am proximalen Ende mit dem distalen Ende des Sockels verbindbar ist und wobei die Lichtquelle (5) am distalen Ende der Sonde (4) positioniert ist.

2. Vorrichtung (1) nach Anspruch 1, **dadurch gekennzeichnet, dass** die Lichtquelle (5) in einem Winkel ß von 0° *bis 90°* zur Längsachse (A) des Schaftes angeordnet ist.

3. Vorrichtung (1) nach einem der Ansprüche 1 oder 2, **dadurch gekennzeichnet, dass** Sockel und Sonde integral sind.

4. Vorrichtung (1) nach einem der Ansprüche 1 bis 3, **dadurch gekennzeichnet, dass** die Vorrichtung (1) eine Schnittstelle aufweist, die mit einer Steuereinrichtung verbindbar ist, über die Wellenlänge, Farbtemperatur, Intensität und/oder Helligkeit des Lichts einstellbar sind.

5. Vorrichtung (1) nach einem der Ansprüche 1 bis 4, **dadurch gekennzeichnet, dass** die Sonde (4) einen Außendurchmesser von maximal 20 bis 30 Gauge aufweist.

6. Vorrichtung (1) nach einem der Ansprüche 1 bis 5, **dadurch gekennzeichnet, dass** ein proximaler Bereich (11) des Schaftes als Handstück (30) zur Navigation des Lichtkegels ausgestaltet ist.

7. Vorrichtung (1) nach einem der Ansprüche 1 bis 6, **dadurch gekennzeichnet dass** im proximalen Bereich des Schaftes (11) eine Halterung (30) angebracht ist, wobei die Halterung (30) de-arretiert und entfernt werden kann.

8. Vorrichtung (1) nach einem der Ansprüche 1 bis 7, **dadurch gekennzeichnet, dass** die Energiequelle (6) eine oder mehrere Batterien, einen oder mehrere Akkus, und/oder einen oder mehrere Kondensatoren umfasst.

9. Vorrichtung (1) nach einem der Ansprüche 1 bis 8, **dadurch gekennzeichnet, dass** die Energiequelle im proximalen Bereich des Schaftes in den Schaft integriert ist oder dass die Energiequelle in einem Gehäuse integriert ist.

10. Vorrichtung (1) nach einem der Ansprüche 1 bis 9, **dadurch gekennzeichnet, dass** die Vorrichtung (1) einen Strombegrenzer aufweist.

11. Vorrichtung (1) nach einem der Ansprüche 1 bis 10, **dadurch gekennzeichnet, dass** die Vorrichtung (1) einen nicht mit dem Schaft verbundenen Instrumentenkanal aufweist, in dem zumindest der distale Bereich des Schaftes rotationsbeweglich geführt wird, wobei der Instrumentenkanal am Schaft von distal nach proximal verläuft und am distalem Ende eine Öffnung für den Austritt des Lichtes aufweist.

12. Vorrichtung (1) nach einem der Ansprüche 1 bis 11, **dadurch gekennzeichnet, dass** die Vorrichtung (1) alle Bestandteile integral umfasst und/oder als Wegwerfartikel ausgebildet ist.

13. Vorrichtung (1) nach einem der Ansprüche 1 bis 12, **dadurch gekennzeichnet, dass** einige oder alle Komponenten a), b), c), d) und e) der Vorrichtung (1) getrennt vorliegen können und zusammensetzbar sind.

14. Vorrichtung (1) nach einem der Ansprüche 1 bis 13, dad fts so ausgebildet ist, dass es beim Überstreichen von Gewebe dieses nicht schädigt.urch gekennzeichnet, dass das distale Ende des Scha

15. Verwendung der Vorrichtung nach einem der Ansprüche 1 bis 14 zur nicht-invasiven, transskleralen oder minimalinvasiven, inokularen Beleuchtung des intraokularen Raumes des menschlichen oder tierischen Auges.

16. Verfahren zur nicht-invasiven, transskleralen oder minimalinvasiven, inokularen Beleuchtung des intraokularen Raumes des menschlichen oder tierischen Auges, wobei das Verfahren die Verwendung gemäß Anspruch 15 umfasst.

Figur 1

EP 3 395 232 A1

Figur 2

EP 3 395 232 A1

Figur 3

Figur 4

Figur 5

Figur 6

Figur 7

Europäisches
Patentamt

European
Patent Office

Office européen
des brevets

# EUROPÄISCHER RECHERCHENBERICHT

**Nummer der Anmeldung**

EP 17 16 8314

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2006 051736 A1 (GEUDER AG [DE]) 8. Mai 2008 (2008-05-08) * Absätze [0038], [0041], [0046] - [0048] * | 1-16 | INV. A61B3/00 |
| | ----- | | |
| A | US 2013/038836 A1 (SMITH RONALD T [US]) 14. Februar 2013 (2013-02-14) * Ansprüche 1,5 * | 1-16 | |
| | ----- | | |
| A | US 2014/288417 A1 (SCHMIDTLIN EDOUARD G [US] ET AL) 25. September 2014 (2014-09-25) * Anspruch 1 * | 1-16 | |
| | ----- | | |
| A | WO 2011/019505 A1 (ALCON RES LTD [US]; PAPAC MICHAEL JAMES [US]; HORVATH CHRISTOPHER [US]) 17. Februar 2011 (2011-02-17) * Anspruch 1 * | 1-16 | |
| | ----- | | |

**RECHERCHIERTE SACHGEBIETE (IPC)**

A61B

Der vorliegende Recherchenbericht wurde für alle Patentansprüche erstellt

| Recherchenort | Abschlußdatum der Recherche | Prüfer |
|---|---|---|
| München | 31. Oktober 2017 | Martelli, Luca |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 16 8314

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-10-2017

| Im Recherchenbericht angeführtes Patentdokument | Datum der Veröffentlichung | Mitglied(er) der Patentfamilie | Datum der Veröffentlichung |
|---|---|---|---|
| DE 102006051736 A1 | 08-05-2008 | DE 102006051736 A1<br>EP 2086389 A1<br>US 2011069278 A1<br>WO 2008052508 A1 | 08-05-2008<br>12-08-2009<br>24-03-2011<br>08-05-2008 |
| US 2013038836 A1 | 14-02-2013 | KEINE | |
| US 2014288417 A1 | 25-09-2014 | US 2014288417 A1<br>WO 2014153379 A1 | 25-09-2014<br>25-09-2014 |
| WO 2011019505 A1 | 17-02-2011 | US 2011037948 A1<br>WO 2011019505 A1 | 17-02-2011<br>17-02-2011 |

EPO FORM P0461

Für nähere Einzelheiten zu diesem Anhang : siehe Amtsblatt des Europäischen Patentamts, Nr.12/82

**EP 3 395 232 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20040004846 A1